# EUROPEAN PATENT APPLICATION

(11) **EP 2 965 735 A1**
(43) Date of publication of application: **13.01.2016**
(21) Application number: 14176556.0
(22) Date of filing: 10.07.2014
(51) Int. Cl.: A61F 13/62

(54) **Fastening tab**

(71) Applicant: 3M Innovative Properties Company, Saint Paul, MN 55133-3427 (US)
(72) Inventor: von Jakusch, Egbert, 42653 Solingen (DE); Tuschy, Joerg, 50181 Bedburg (DE); Kitzer, Peter, 6101 BZ Echt (NL)
(74) Representative: Gabriel, Kiroubagaranne

(57) **Abstract**

A fastening tab for an absorbent article, the fastening tab comprising a proximal end and a distal end, said proximal end comprising a backing including a first major surface and a second major surface, wherein said first major surface is provided with a release surface, wherein the fastening tab further comprises a mechanical fastening patch, said patch comprising on one side male fastening elements and on the opposite side an adhesive, wherein the patch is releasably attached to the release surface via the adhesive, said distal end comprising a backing including a first major surface and a second major surface, wherein said first major surface has a structure or is provided with a structure that is adapted to be releasably engaged by said male fastening elements of the patch. Fastening tapes comprising a plurality of such fastening tabs, wherein the fastening tabs are arranged in an endless form on the tape so that individual fastening tabs can be provided from the fastening tape by cutting the tape across its width and rolls of such fastening tapes.

## Description

### Field

The present application relates to fastening tabs for disposable, absorbent articles, fastening tapes and rolls of fastening tape including such fastening tabs as well as absorbent articles comprising fastening tabs and methods of manufacturing and using such absorbent articles.

### Background

Disposable, absorbent articles, such as diapers and incontinence briefs, are widely used not only in the sector of child care, but also for adults suffering from incontinence. The absorbent articles are typically reclosable by fastening tabs that are generally easy to handle and provide reliable fixation.

Generally such fastening tabs either comprise an adhesive-based closure mechanism or a mechanical-based closure mechanism or a combination of both. Fastening tabs relying on a mechanical closure mechanism typically comprise hook-like fastening elements whereby the tab is normally attached to the rear waist portion of the diaper or incontinence brief or, if applicable, to an ear thereof. A large number of documents described such fastening tabs including among others for example EP 2 103 293, WO 2000/013641, and EP 2 055 283. For closing diapers or incontinence briefs provided with such fastening tabs, the fastening tab is typically fastened to a landing zone provided on the front waist portion of the diaper or incontinence brief, respectively. For this purpose, the landing zone typically comprises a surface with a loop material being adapted to engage the hooks of the fastening tab. The provision of such a landing zone may be omitted, when the back sheet of the diaper or the incontinence brief is made of a material that is suitable to engage with hooks of the fastening tab or when the fastening tab includes a so-called loop-transfer element, where upon during initial use of the tab, a piece of loop-comprising material engaged to the hooks on the fastening tab is transferred from the tab onto the front portion of diaper or incontinence brief, as the case may be. Documents describing loop-transfer tabs or tapes include among others US 5,383,872, EP 1 449 505 and EP 1 000 598.

### Summary of the Invention

Surprisingly it has been found that by providing a fastening tab with a transferrable mechanical fastening patch including male fastening elements (rather than loop material) that a tab is obtained that in conjunction with a diaper or incontinence brief has a greater degree of adjustability and/or ease in use. This is particularly surprising since although loop-transfer tabs have been commercially known and used since at least a decade, the application of fastening tabs including a transferrable patch of male fastening elements has not been commercially explored. Furthermore, it has been found that the overall construction of such tabs can be simplified in comparison to known loop transfer tabs, in particular so simplified that desirably stable rolls of fastening tapes towards production of such tabs can be provided.

Accordingly, in one aspect of the present invention there is provided a fastening tab comprising a proximal end and a distal end. The proximal end comprises a backing including a first major surface and a second major surface, wherein said first major surface is provided with a release surface (such as a low surface energy film, a microstructured film, or a low adhesion backside coating), wherein the fastening tab further comprises a mechanical fastening patch, said patch comprising on one side male fastening elements and on the opposite side an adhesive, wherein the patch is releasably attached to the release surface via the adhesive. The distal end comprises a backing including a first major surface and a second major surface, wherein said first major surface has a structure or is provided with a structure that is adapted to be engaged by said male fastening elements of the patch.

To further facilitate greater degrees of adjustability and/or ease in use, it has been found advantageous to provide tabs wherein the width of the engaging-structure (e.g. having regard to the transverse direction of the tab from the proximal end to the distal end) is favorably at least 2 times greater than the width of the male fastening element comprising patch, more favorably the width of the engaging-structure is at least 2.5 times greater than the width of the of male fastening element comprising patch, most favorably the width of the engaging-structure is at least 3 times greater than the width of the of male fastening element comprising patch.

Desirably the proximal and distal ends of the tab are configured and arranged such that at least a portion of one end (in particular the distal end) can be folded onto the other end (in particular the proximal end) so that said male fastening elements can releasably engage said engaging-structure.

Desirably the fastening tab is configured and arranged, such that the force to disengage the male fastening elements from the engaging-structure is higher than the force to detach the adhesive of the male fastening element patch from the release surface (such as a low adhesion backside). It will be recognized that through the use of the release surface (e.g. a low adhesion backside), favorably the force to detach the adhesive of the male fastening element comprising patch from the release surface will be typically low, thus facilitating the desirable configuration and arrangement towards a fastening tab where the force needed to disengage the male fastening elements from the engaging-structure will be higher.

The adhesive of the male fastening element comprising patch, referred to in part in the following as the first adhesive, is favorably a pressure sensitive adhesive, more favorably pressure sensitive adhesives based on styrene-isoprene-styrene block copolymer/tackifier mixtures. First adhesives may have a coating weight in the range of 20 to 50 g/m². Alternatively or additionally, First adhesive may have a thickness in the range of 20 microns to 60 microns.

As described in more detail below, when using fastening tabs as described herein with absorbent articles, the male fastening element patch will be moved from the tab to the absorbent article, such that the first adhesive will be adhered to a portion of the absorbent article, e.g. the front waist portion of a diaper or an incontinence brief. Desirably the fastening tab is configured and arranged, such that the force to detach the first adhesive of the transferred male fastening element patch from the absorbent article is greater than force needed to disengage the male fastening elements from the engaging-structure. In regard to the latter, it will be appreciated by the skilled reader that forces needed to disengage common commercial "hook-and-loop" pairings are typically around 5 N /25 mm.

Favorably the first adhesive has a 90° peel adhesio n to a smooth polyethylene test surface having an average surface roughness value Rₐ of about 1.4 µm and an average peak to valley height value R_{z} of about 12.5µm greater than 5 N/25 mm, in particular equal to or greater than 6 N/25 mm, more particularly equal to or greater than 7 N/25 mm e.g. as measured according to FINAT Test Method No. 2 "Peel Adhesion (90°) at 300 mm per minute" in 5^{th} Edition FINAT Technical Handbook (Test Methods) 1999 using an automatic roller with a roll-down weight of 5,000 g and roller speed 5 mm/sec, where after placing the sample to be tested on the polyethylene test surface, the sample is rolled down once in each direction . Within the aforesaid range, while first adhesives having 90°peel adhesi ons greater than 15 N/25 mm may be used, generally first adhesives having 90°peel adhesions up to and including 15 N/25 mm are suitable. In addition or alternatively, the first adhesive favorably has a tack value to a smooth polyethylene test surface having an average surface roughness value Rₐ of about 1.4 µm and an average peak to valley height value R_{z} of about 12.5µm equal to or greater than 5 N/25 mm, in particular equal to or greater than 6 N/25 mm, more particularly equal to or greater than 7 N/25 mm e.g. as measured according to FINAT Test Method No. 9 "'Loop' tack measurement" in 5th Edition FINAT Technical Handbook (Test Methods) 1999. It will be appreciated that samples tested in the aforesaid peel adhesion and/or tack test method comprise the first adhesive as provided on the male fastening element comprising patch (e.g. first adhesive on one major surface of the sample to be tested and male fastening elements on the opposite major surface thereof).

Desirably the second major surface at the proximal end is provided at least in part with a second adhesive. The second major surface at the distal end may also be provided at least in part with a third adhesive. In the event, second and third adhesives are provided they may be identical or different. The backing of the proximal end and the backing of the distal end may be either integral or two separate elements, wherein in the latter case the two separate elements are favorably connected to another via a connection member.

The male fastening elements are favorably selected from the group consisting of hook fasteners, mushroom-shaped fasteners, stem-shaped fasteners, cup-shaped fasteners, T-shaped fasteners and mixtures thereof.

The engaging-structure may comprise a structure selected from the group consisting of nonwoven structures, loops, mushroom-shaped fasteners, cup-shaped fasteners, T-shaped fasteners and mixtures thereof.

In another aspect of present invention there is provided a fastening tape comprising a plurality of fastening tabs as described herein, wherein the fastening tabs are arranged in an endless form on the tape so that individual fastening tabs can be provided from the fastening tape by cutting the tape across its width. In a further aspect of the present invention there is a provided a roll of such fastening tape.

Desirably, the proximal and distal ends of the fastening tabs are configured and arranged in the roll such that said male fastening elements are not engaging said engaging-structure. In other words, the fastening tape is desirably rolled in a flat, unengaged (e.g. unfolded) configuration. Moreover relative to the width of the tape as provided in the roll, the proximal and distal ends of the fastening tabs are positioned favorably in two outer lateral regions of rolled fastening tape. This is advantageous in that across the width of the tape the overall height of the tape on the roll is relatively low (e.g. in comparison to known loop transfer tapes where the loop and hooks are typically provided in engaging (e.g. folded) configuration) and the highest elements in the outer two lateral regions of the rolled tape (in other words the male fastening elements and the engaging-structure in the proximal and distal ends, respectively, of the endless configured fastening tabs on the tape) are not too dissimilar in height; both of the aforesaid contributing favorably to the provision of stable rolls.

The ends of the male fastening elements which are distal to the first adhesive (i.e. those ends of the male fastening elements typically involved in the releasable engagement to the engaging-structure) are favorably positioned in the roll towards the second major surface at the proximal end. For those embodiments wherein the second major surface at the proximal end is provided at least in part with a second adhesive and said ends of the male fastening elements are in contact with said second adhesive in the roll, this will further facilitate the stability of the roll, and surprisingly without detrimentally effecting the unwinding of the roll. Moreover, it has been observed even if the force (e.g. peel force) to disengage said ends of the male fastening elements from the second adhesive is higher than the force needed to detach the first adhesive of the male fastening element patch from release surface (which is normally the case), the roll can be unwound without the first adhesive and release surface peeling apart. Without wanting to be bound to a particular theory, this seems to be due to a combination of factors: one being that the tabs are arranged in an endless form on the roll and thus there is no leading edge from which a peeling of the first adhesive from the release surface can be initiated, and another being that the ends of individual male fastening elements that are in contact with second adhesive typically have edges from which a peeling of male fastening elements from the second adhesive can be initiated.

Accordingly rolls described herein are favorably free of a release liner between the male fastening elements and the second major surface at the proximal end, and in particular for those embodiments having a second adhesive at the proximal end, are free of a release liner between the male fastening elements and the second adhesive provided on said second major surface at the proximal end. This is particularly advantageous in light of known loop-transfer tapes which commonly employ a release liner in the roll. Rolls are also favorably free of a release liner between the engaging-structure and the second major surface at the distal end, and in particular for those embodiments including a third adhesive at the distal end, are free of a release liner between the engaging structure and the third adhesive provided on said second major surface.

In a further aspect of the present invention, there is provided an absorbent article, in particular a diaper or an incontinence brief, comprising a back sheet, a top sheet and an absorbent core there between and a fastening tab as described herein, wherein the fastening tab is attached to the absorbent article at least its proximal end.

For absorbent articles that are diapers and incontinence briefs, fastening tabs may be attached to a rear waist portion or alternatively for absorbent articles comprising an ear or ears, at a distal end portion of the ear(s), more particularly to the inside of the rear waist portion or to the inside of the ear(s), respectively.

Favorably prior to use by the user, e.g. in the packaged and/or storage condition of the absorbent article, the fastening tab is folded so that the male fastening elements are releasably engaged with the engaging-structure, more favorably the distal end of the fastening tab is folded onto the proximal end of the fastening tab so that the male fastening elements are releasably engaged with the engaging-structure.

As mentioned above, as the result of the use of a release surface in the proximal end of the fastening tab, typically the force to detach the adhesive of the patch from the release surface is lower, in particular much lower, than the force to disengage the male fastening elements from the engaging-structure. This means that starting from for example the favored packaged configuration of an absorbent article, e.g. a diaper or an incontinence brief, where the fastening tab is folded and the male fastening elements are releasably engaged with the engaging-structure, when the user unfolds the fastening tab, the adhesive of the patch will detach from the release surface while the male fastening elements of the patch remain releasably engaged with the engaging-structure at the distal end of the tab. Accordingly the patch with its engaged fastening elements is transferred from the proximal end onto the distal end of the fastening tab and additionally the adhesive of the patch is now exposed.

After this first unfolding, the user can then close the absorbent article, e.g. a diaper or an incontinence brief, by pressing the distal end of the fastening tab onto a respective (e.g. appropriate) portion of the absorbent article, so that the adhesive of the patch adheres to the respective portion of the absorbent article. For example for absorbent articles that are diapers or incontinence briefs, the adhesive of the patch can be adhered e.g. on a front waist portion, in particular on the outside of the front waist portion. Upon this first closure the adhesive of the patch with its male fastening elements will be advantageously affixed onto the absorbent article, i.e. onto said respective portion of the absorbent article. Thus in this sequence of unfolding and first closure, the patch has been transferred from the proximal end to the distal end of the fastening tab and subsequently from the distal end of the fastening tab onto the respective portion of the absorbent article.

Furthermore, it will be recognized that after the aforementioned second transfer, the patch and the fastening tab will favorably function like a regular mechanical-based closure system. Moreover upon the user pulling the fastening tab to open the absorbent article, the engaging-structure at the distal end of the fastening tab will disengage from the fastening elements of the patch, while the patch remains adhered to the absorbent article. Thereafter the user may then reclose the absorbent article by pressing the distal end of the fastening tab onto the patch such that the engaging-structure at the distal end of the fastening tab releasably (re-)engages the fastening elements of the patch.

It is to be understood that the present invention covers all combinations of particular, suitable, desirable, favorable advantageous and preferred aspects of the invention described herein.

### Brief Description of Drawings

The invention will now be described with reference to the following drawings.
Figure 1 represents a cross-sectional, schematic illustration of an exemplary fastening tab in accordance to the invention described herein.
Figure 2A represents a cross-sectional schematic illustration of a further exemplary fastening tab in accordance to the invention described herein.
Figure 2B represents an isometric view of a exemplary roll of fastening tape in accordance to the invention described herein, said exemplary roll including a plurality of fastening tabs as shown in Figure 2A in an endless form.
Figure 3A represents a cross-sectional schematic illustration of another exemplary fastening tab in accordance to the invention described herein.
Figure 3B represents a cross-sectional schematic illustration of yet another exemplary fastening tab in accordance to the invention described herein
Figure 4 represents a cross-sectional schematic illustration of an additional exemplary fastening tab in accordance to the invention described herein.
Figure 5A represents an isometric view of an exemplary absorbent article, in particular a diaper or an incontinence brief, in accordance to the invention described herein, said absorbent including the two fastening tabs as shown in Figure 1, while Figure 5B represents a cross-sectional schematic illustration of a portion of said absorbent article showing said tabs.
Figure 6A represents an isometric view of the exemplary absorbent article shown in Figure 5 where the absorbent article is a desired folded configuration for e.g. storage, while Figure 6B represents a cross-sectional schematic illustration of a portion of said folded absorbent article showing the fastening tabs in a folded configuration.
Figure 7A represents an isometric view of the exemplary absorbent article shown in Figures 5 and 6 where the absorbent article is shown in a configuration after unfolding for e.g. use, while Figure 7B represents a cross-sectional schematic illustration of said unfolded absorbent article.
Figure 8A represents an isometric view of the exemplary absorbent article shown in Figure 5 to 7 where the absorbent article is shown in a configuration after unfolding and prior to a first fastening, while Figure 8B represents a cross-sectional schematic illustration of said absorbent article.
Figure 9A represents an isometric view of the exemplary absorbent article shown in Figures 5 to 8, where the absorbent article is shown in a fastened configuration, while Figure 9B represents a cross-sectional schematic illustration of said fastened absorbent article.
Figure 10A represents an isometric view of the exemplary absorbent article shown in Figures 5 to 9 where the absorbent article is shown in a configuration after un-fastening, while Figure 10B represents a cross-sectional schematic illustration of said un-fastened absorbent article.
Figure 11A represents an isometric view of the exemplary absorbent article shown in Figures 5 to 10 where the absorbent article is shown in a configuration after re-fastening, while Figure 11 B represents a cross-sectional schematic illustration of said re-fastened absorbent article.
Figures 12A represents an isometric view of another exemplary absorbent article, in particular a diaper or incontinence brief, in accordance to the invention described herein, said absorbent including the two fastening tabs as shown in Figure 3 wherein Figure 12B represents an isometric view of the absorbent article shown in Figure 12A, where the tabs are shown in a desired folded configuration and Figure 12C represents an isometric view of the absorbent article shown in Figures 12A and B where the absorbent article is in a desired folded configuration.

In the description that follows, unless expressly stated otherwise, terms such as 'top', 'bottom', 'above', 'below', etc, refer only to features as shown in the Figures, and no restriction as to orientation of use, etc, is intended. Not all Figures are to the same scale.

### Detailed Description

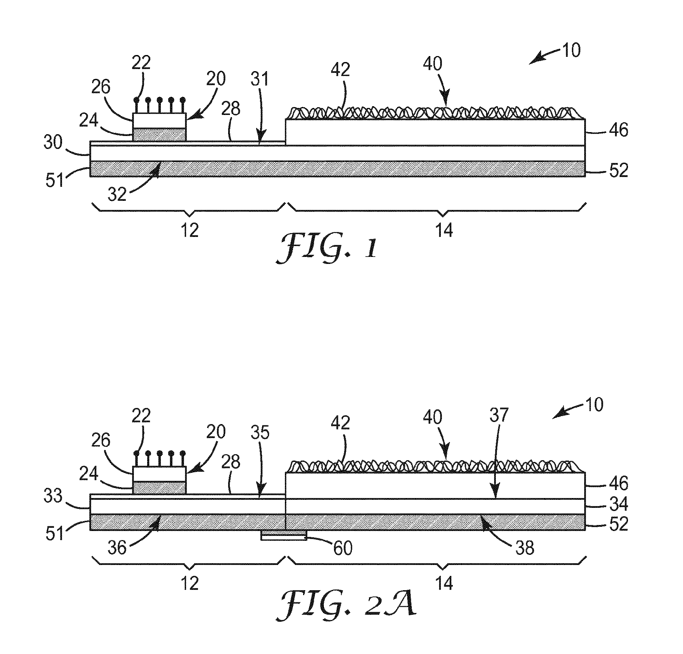

Figure 1 represents a cross-sectional, schematic illustration of an exemplary fastening tab for an absorbent article, in particular a disposable absorbent article, in accordance with the invention described herein. The fastening tab 10 includes a proximal end 12 and an opposite distal end 14 and comprises a backing 30 having a first major surface 31 and a second major surface 32. It will be appreciated that the backing is integral over the proximal and distal ends. In use in conjunction with an absorbent article, typically the proximal end (which could also be termed the manufacturer's end) is positioned towards the interior of the article while the distal end (which could also be termed the user's end) is positioned away from the interior of the article. In the proximal end, the first major surface is provided with a release surface 28, in particular a low adhesion backside coating. The fastening tab also includes a mechanical fastening patch 20, said patch comprising on one side male fastening elements 22 of a mechanical fastening system and on the opposite side an adhesive 24 which is typically provided on a substrate 26, wherein the patch is releasably attached to the release surface via the adhesive. As mentioned above this adhesive is referred to for the most part herein as the first adhesive. It will be appreciated that the entire first major surface in the proximal end may be provided with a release surface as shown in this embodiment, or alternatively just that portion of the first major surface to which the fastening patch is releasably adhered may be provided with a release surface. As mentioned above, the first major surface of the distal end has a structure or is provided with a structure that can be releasably engaged by said male fastening elements of the fastening patch, said structure being referred to as the engaging-structure. In the exemplary embodiment depicted in Figure 1, the first major surface is provided with an engaging-structure 42. In particular, a strip 40 including the engaging-structure 42 on a substrate 46 is affixed to the first major surface in the region of the distal end. As shown in this embodiment the engaging-structure may be provided across the whole width of the distal end or alternatively the engaging-structure may only be provided across a portion thereof.

In the exemplary embodiment depicted in Figure 1, the second major surface at the proximal end as well as at the distal end is provided with a second adhesive 51 and a third adhesive 52, respectively for affixing the tab onto an absorbent article. In this illustrated embodiment the second and third adhesives are identical whereby the adhesive coating is an integral layer. It will be appreciated that in this embodiment as well as the other embodiments described herein, the entire portion of a respective surface may be provided with the respective adhesive e.g. in the form as a continuous coating, or alternatively just a part thereof may be provided with adhesive, e.g. adhesive may be provided in the form of a pattern coating.

Adhesives suitable for use as second and/or third adhesives include pressure sensitive adhesives, more favorably pressure sensitive adhesives based on styrene-isoprene-styrene block copolymer/tackifier mixtures. Second and/or third adhesives may have coating weights in the range of 20 to 50 g/m². Alternatively or additionally, second and/or third adhesives may have thicknesses in the range of 20 microns to 100 microns.

Second and/or third adhesives, like first adhesives, may favorably have a 90° peel adhesion to a smooth polyethylene test surface having an average surface roughness value Rₐ of about 1.4 µm and an average peak to valley height value R_{z} of about 12.5µm greater than 5 N/25 mm, in particular equal to or greater than 6 N/25 mm, more particularly equal to or greater than 7 N/25mm e.g. as measured according to FINAT Test Method No. 2 "Peel Adhesion (90°) at 300 mm per minute" in 5th Edition FINAT Technical Handbook (Test Methods) 1999 using an automatic roller with a roll-down weight of 5,000 g and roller speed 5 mm/sec, where after placing the sample to be tested on the polyethylene test surface, the sample is rolled down once in each direction. Within the aforesaid range, while second and/or third adhesives having 90° peel adhesions greater than 15 N/ 25 mm may be used, generally 90°peel adhesions up to and including 15 N/ 25 mm are suitable.

In the peel adhesion and loop tack testings mentioned herein, the polyethylene test surface may be provided by adhering (e.g. via double sided tape) an appropriate polyethylene film onto a (e.g. glass or stainless) test plate. Such a polyethylene film may be obtained as follows: A low density polyethylene resin (such as the low density resin marketed under the trade designation TENITE 1550P by Eastman Chemical Co. Kingsport, Tennessee) having a melt index of 3.5 grams/10 min and a density of 0.918 grams/cm is extruded at a melt temperature of 182°C vertically downward through a conventional coat-hanger slot extrusion die. The melt exiting the die is drawn into a nip formed by a mirror finish chrome roll (8°C inlet water temperature) and a silicone rubber roll (7°C inlet water temperature) resulting in a film of 330 microns thickness, whereby the surface of the film that was in contact with the chrome roll will be that surface of the film that used for testing. Said surface typically has an average surface roughness value Rₐ of 1.4µm and an average peak to valley height value R_{z} of about 12.5µm as measured using a laser profilometer (e.g. a laser profilometer model number UB-16 available from UBM Messtechnik GmbH, Ettlingen, Germany) and computed in accordance with Deutsche Industrie Norm (DIN) 4768 and DIN 4762.

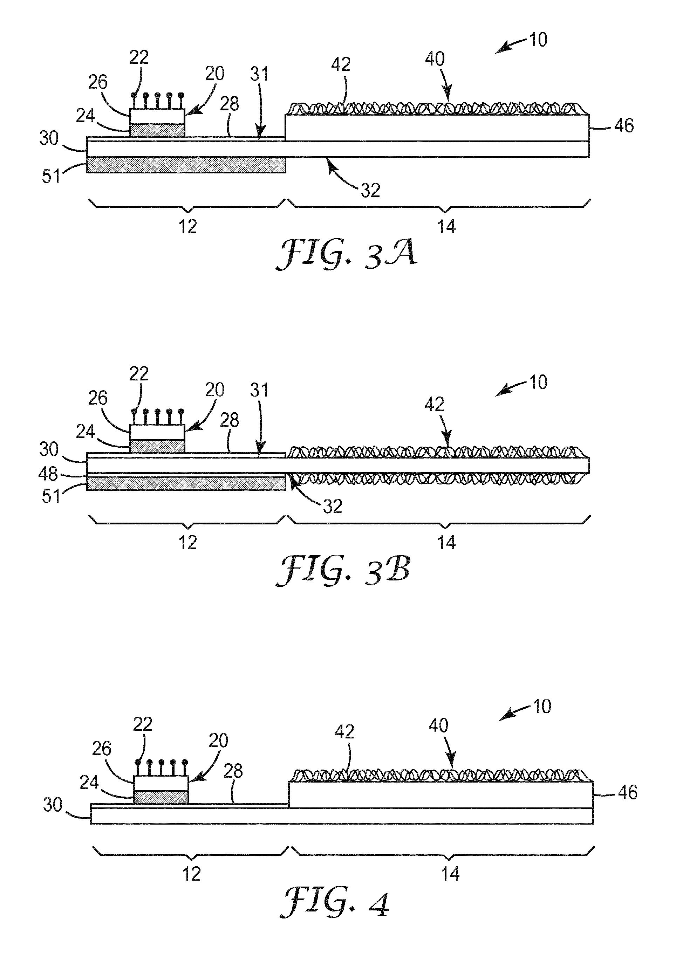

In alternative embodiments, the second major surface at just the proximal end may be provided with an adhesive for affixing the tab onto an absorbent article. Such an embodiment in depicted in Figure 3A, where the construction is the same as the embodiment in Figure 1 including a second adhesive 51 on the second major surface 32 at the proximal end 12, but now with no adhesive on the second major surface at the distal end 14. In the other embodiments, the second major surface at both the proximal and distal ends may be free of adhesive. Figure 4 shows such an exemplary embodiment, where the construction is the same as the embodiment in Figure 1 except no adhesive is provided on the second major surface of the backing. Such tabs may be affixed at least at their proximal ends to absorbent articles through non-adhesive means, such as ultrasonic or hot air bonding, or via adhesive provided onto the tab and/or the absorbent article just at the time of affixing the tab onto the absorbent article.

Figure 2A provides a further alternative exemplary embodiment in accordance with the invention described herein. This embodiment is similar to that shown in Figure 1, except for several structure differences. One major difference is that the fastening tab 10 includes two separate backings, one 33 in the proximal end 12 and the other 34 in the distal end 14, each having a first major surface 35,37 and a second major surface 36,38 respectively. The second major surface 36 of the backing 33 at the proximal end 12 is provided with a second adhesive 51, while the second major surface 38 of the backing 34 at the distal end 14 is provided with a third adhesive 52. The second and third adhesives may be identical or different. It will be appreciated that in comparison to the previously described embodiments, the exemplary embodiment shown in Figure 2A consists of two separate elements - one forming the proximal end and the other forming the distal end - where the two elements are joined by a connection member 60. In this embodiment the connection member bridges across the second and third adhesives. It will be appreciated that for alternative two-backing embodiments which include for example no adhesive on the second major surfaces or only include a second adhesive on the second major surface of the proximal-end-backing, the connection member may then be a connection member which bridges across the backings at their second major surface or the distal end backing at its second major surface and the proximal end second adhesive, respectively. Such two-element embodiments, in particular tapes comprising such tabs, can be favorable in that they can be created with ease by joining separate "hook" and "loop" tape materials together via an appropriate connection member on the non-fastening sides thereof.

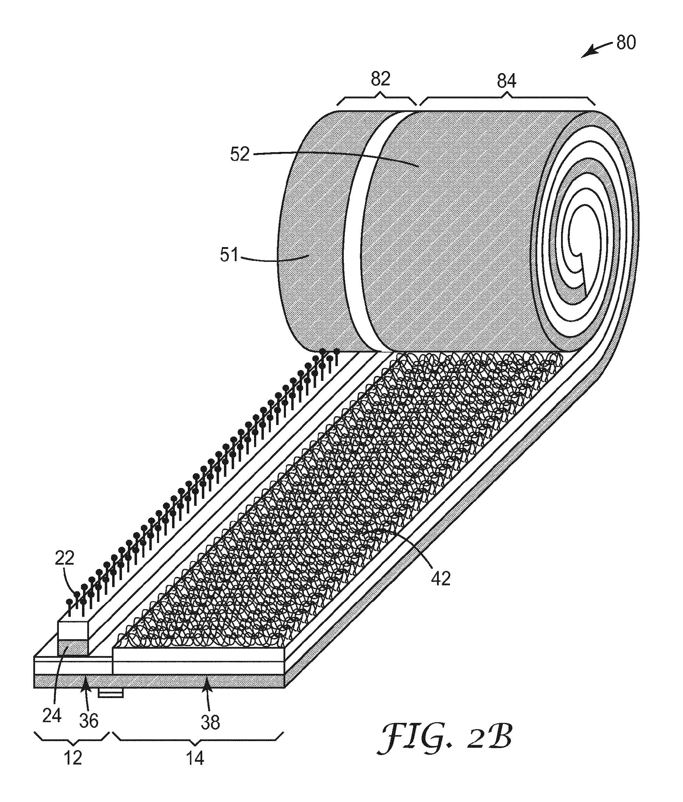

As indicated *supra,* the present invention further relates to tapes comprising a plurality of fastening tabs as described herein as well as rolls of tapes comprising a plurality of fastening tabs. An exemplary embodiment of such tape and roll is shown in Figure 2B. In particular Figure 2B provides a schematic perspective view of a fastening tape 80 including fastening tabs of the type shown in Figure 2A. The fastening tabs are arranged in endless form on the tape 80 so that individual tabs can be cut from the fastening tape by cutting the tape across its width. As can be appreciated from Figure 2B, desirably on the roll the male fastening elements 22 are not engaged to the engaging-structure 42. In other words the tape is desirably rolled in a flat (e.g. unfolded) un-engaged configuration. Moreover, as can be appreciated from Figure 2B relative to the width of the tape as provided in the roll, the proximal and distal ends 12, 14 of the endless configured fastening tabs are desirably positioned in two outer lateral regions 82, 84 of rolled fastening tape. Furthermore, in the roll, the ends of the male fastening elements 22 distal to the first adhesive 24 are favorably positioned towards the second major surface 36 at the proximal end 12, and in this particular exemplary embodiment thus in contact with the second adhesive 51. As illustrated in this exemplary embodiment, the roll is advantageously free of a release liner between the male fastening elements 22 and the second major surface 36 at the proximal end 12, in particular between the male fastening elements and the second adhesive 51 provided on said second major surface at the proximal end. Also it will be appreciated that the roll is favorably free of a release liner between the engaging-structure 42 and the second major surface 38 at the distal end 14, in particular between the engaging structure and the third adhesive 52 provided on said second major surface.

The backing of fastening tabs described herein may be selected from a variety of films or sheetings including single-layered and multi-layered films, co-extended films, laterally laminated films or films comprising film layers. The layers of such films or sheetings may comprise various materials such as, for example, polypropylene, polyvinylchloride, polyethylene terephthalate, polyethylene, polyolefin copolymers or blends of polyolefins such as, for example, a blend of polypropylene, low density polyethylene and/or linear low density polyethylene, textiles, and nonwoven and foamed materials. In alternate embodiments, the backing of fastening tabs described herein may be selected from a variety of fibrous webs, including wovens, knitted webs or nonwovens. For embodiments including a fibrous web as a backing and a second and/or third adhesive, the backing may be poly-coated to minimize penetration of adhesive into the backing. Fibrous-web-backings (e.g. nonwoven backings) may be favorably selected such that the male fastening elements can releasely engage directly the first major surface of the backing, i.e. said first major surface has an inherent structure that can be releasably engaged by said male fastening elements of the patch.

Figure 3B illustrates an exemplary embodiment including a fibrous web as a backing. The construction of the exemplary fastening tab 10 is similar to the exemplary embodiment in Figure 3A, where the male fastening elements 22 comprising patch 20 having the same configuration as in Figure 3A is attached onto a release surface 28 provided on the first major surface 31 at the proximal end 12 and where the second major surface 32 at the proximal end is provided with adhesive 51 for affixing the tab onto an absorbent article (second adhesive). The exemplary embodiment of Figure 3B differs from that shown in Figure 3A in that the backing 30 is a fibrous web (e.g. a nonwoven) and thus the major surfaces of the backing have an inherent fibrous structure. This can be appreciated in Figure 3B, where it can be seen that the first and second major surfaces (31, 32, respectively) at the distal end 14 of the fastening tab show a fibrous structure. In particular, the particular fibrous web backing is advantageously selected such that inherent fibrous structure of at least the surface of the backing subsequently forming the first major surface of the tab can be releasably engaged by the male fastening elements of the patch, so that the inherent fibrous surface structure of the backing 30 forming the first major surface 31 of the distal end 14 of the tab 10 is the engaging structure 42. It will be appreciated that this can be advantageous in that a separate, additional structural element like the strip 40 in Figure 3A including an engaging structure need not be provided. In the regard to the proximal end 12 of the exemplary fastening tab of Figure 3B, it will be recognized that the release surface 28, e.g. provided in the form a low adhesion backside coating or a low energy release film, covers the fibrous surface structure on the first major surface at the proximal end. Further to prevent second adhesive 51 seeping into the fibrous structure of the backing at the proximal end, a poly-coat 48 is provided between the second adhesive and the second major surface at the proximal end.

The thickness of the backing is favorably between 30 microns and 500 microns, and more favorably between 40 and 150 microns. The basis weight of the backing is favorably between 15 and 500 g/m², more favorably between 20 and 300 g/m² and particularly favorably between 20 and 200 g/m². It will be appreciated by the skilled reader that for a particular basis weight, the thickness of a nonwoven backing will generally greater than a film backing. Having regard to the aforesaid favorable basis weights for backings, nonwoven backings most favorably have basis weights between 35 and 85 g/m².

Substantially any thermoplastic materials suitable for film production can be used to produce the one or more patches of fastening material. Preferred thermoplastic resins include polyesters, such as poly (ethylene terephthalate); polyamides, such as nylon; poly (styrene - acrylonitrile); poly (acrylonitrile - butadiene - styrene); polyolefins, such as polypropylene and/or polyethylene; and plasticized polyvinylchloride.

The male fastening elements may comprise male fastening elements selected from the group consisting of hook fasteners, mushroom-shaped fasteners, stem-shaped fasteners, cup-shaped fasteners, T-shaped fasteners and mixtures thereof. Male fastening elements usually comprise a stem supported by the exposed major surface of a patch of fastening material and an enlarged section which is positioned at the end of the stem opposite to the exposed major surface. The fastening elements can also be formed by stem having no enlarged section at the end of the stem, wherein such stems are preferably essentially conical, cylindrical or pyramidal. Fastening elements suitable in the present invention can be manufactured from a wide range of materials including thermoplastic polymers such as, for example, nylon, polyester, polyolefins (e.g. polypropylene and/or polyethylene) or any combination of these. The fastening elements preferably comprise the material of which the patch of fastening material is formed. The height of the fastening elements may range from 0.1 to 0.4 mm.

Engaging-structures suitable in the present invention can be manufactured from a wide range of materials including thermoplastic polymers such as, for example, nylon, polyester, polyolefins (e.g. polypropylene and/or polyethylene) or any combination of these. As will be described in more detail below, the proximal and distal ends of the tab are advantageously configured and arranged such that at least a portion of one end (in particular the distal end) can be folded onto the other end (in particular the proximal end) so that said male fastening elements can releasably engage said engaging-structure. Depending on the particular male fastening elements used, the engaging-structure may comprise a structure selected from the group consisting of nonwoven structures, loops (e.g. knitted loops, woven loops, extrusion bonded loops), mushroom-shaped fasteners, cup-shaped fasteners, T-shaped fasteners and mixtures thereof. The height of engaging-structure may range up to 0.4 mm.

Desirably, the fastening tab is configured and arranged, such that the force to disengage the male fastening elements from the engaging-structure is higher than the force to detach the adhesive of the fastening element patch from the release surface.

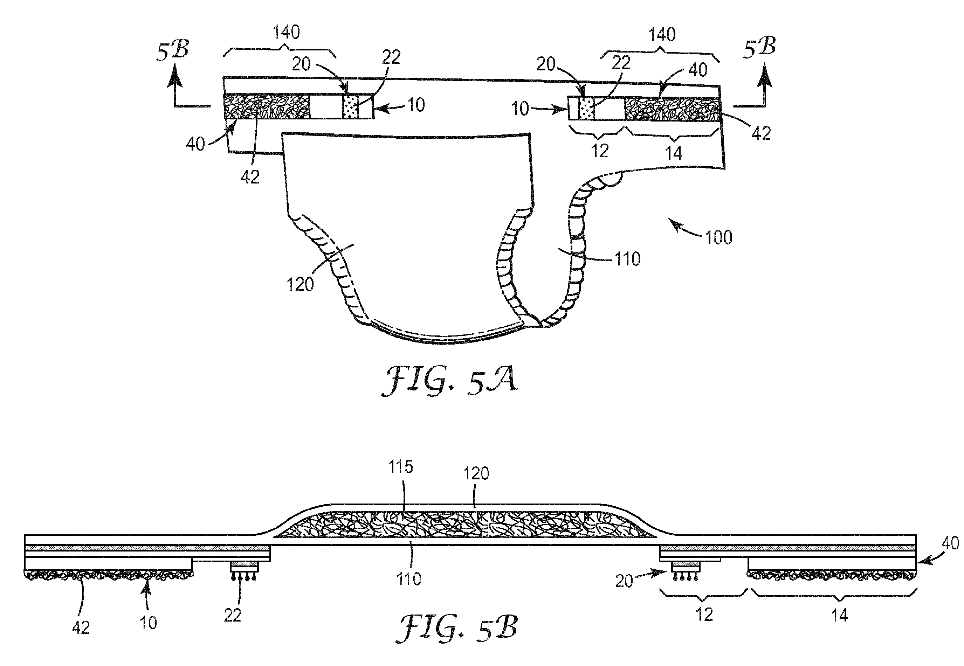

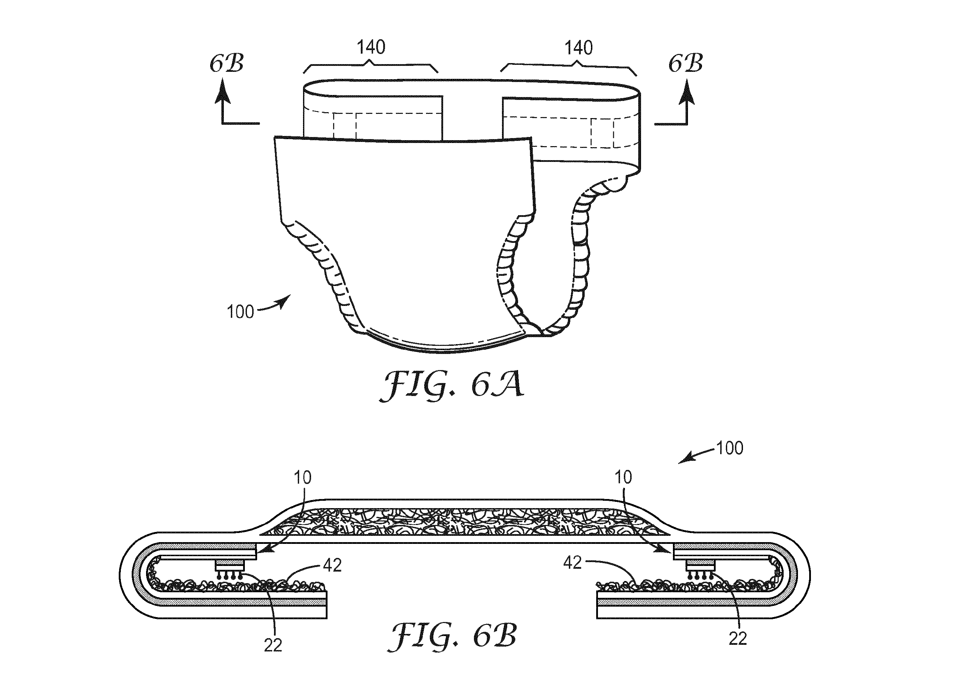

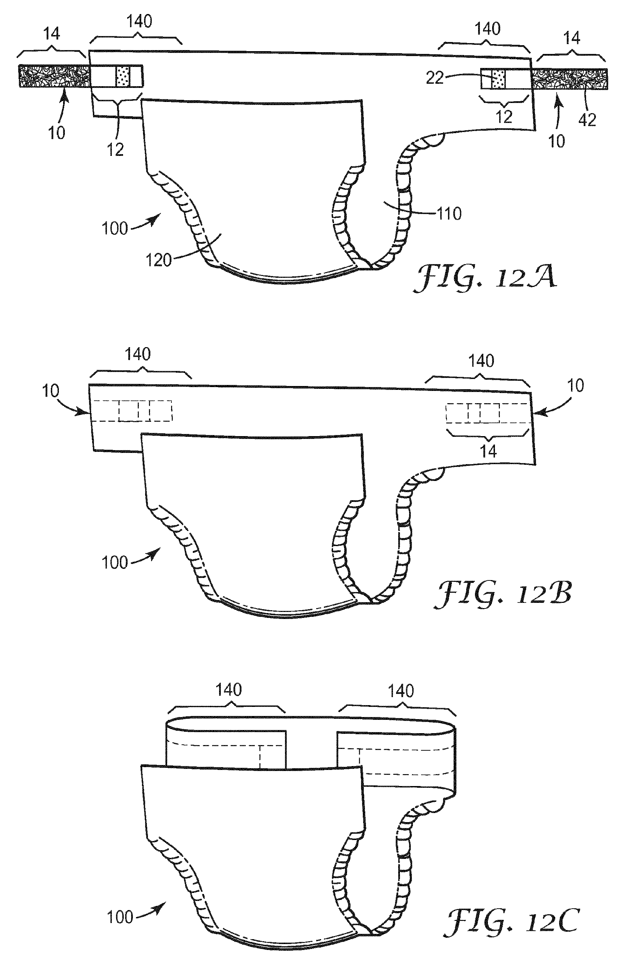

Figures 5 and 6 represent isometric and cross sectional views of an exemplary absorbent article, in particular a disposable absorbent article, more particularly a diaper or an incontinence brief, including two fastening tabs as shown in Figure 1 in exemplary configurations corresponding to a configuration just after attachment of the tabs and a folded configuration for storage and/or packaging, respectively. Figures 12A through 12C represent isometric views of an exemplary absorbent article, in particular a disposable absorbent article, more particularly a diaper or an incontinence brief, including two fastening tabs as shown in either Figure 3A or Figure 4 in configurations corresponding to a configuration just after attachment of the tabs and folded configurations.

In particular, Figures 5A and B shows an isometric and cross sectional view a diaper or an incontinence brief 100 comprising a back sheet 120, a top sheet 110 and an absorbent core 115 (only visible in Figure 5B) there between and two fastening tabs 10 of the type shown in Figure 1. The two fastening tabs 10 are affixed to rear waist portion of the absorbent article, in particular to the inside of the rear waist portion. Moreover, as can be appreciated from Figure 5A, the diaper or incontinence brief includes two ears 140, where the fastening tabs are affixed to the inside (e.g. to the top sheet) of the ear at at least the distal end portion of the ear. In the illustrated embodiment the fastening tabs are affixed across their entire width (i.e. over both the proximal and distal ends 12, 14) onto the top sheet. Figures 5A and B show views of the article for example in a configuration just after attachment of the fastening tabs to the article, whereby the male fastening element 22 comprising patch 20 at the proximal end of the tab is located towards the interior of the article and the engaging-structure 42 comprising strip 40 at the distal end of the tab is located away from the interior of the article. Desirably the distal end of each fastening tab is folded onto the proximal end of the fastening tab so that the male fastening elements are releasably engaged with the engaging-structure. This is for example shown in Figures 6A and B. In particular as can be seen in Figure 6A, the ears 140 of the diaper or incontinence brief 100 are folded towards the interior of the article e.g. for storage and/or packaging purposes. From Figure 6B, it can be seen that with this folding of the ears, the fastening tabs 10 are correspondingly folded so that the male fastening elements 22 of each tab are releasably engaged with the engaging-structure 42.

Figures 12A to C show isometric views of a diaper or an incontinence brief 100 comprising a back sheet 120, a top sheet 110 and an absorbent core (not visible; 115) there between and two fastening tabs of the type shown in either Figure 3 or Figure 4. As can be appreciated from Figure 12A, the diaper or incontinence brief includes two ears 140, where one fastening tab is affixed to the inside (e.g. to the top sheet) of each ear, at least at the distal end portion of the each ear. In this illustrated embodiment, just the proximal ends 12 of the fastening tabs are affixed to the absorbent article so that the distal ends 14 of the fastening tabs extend beyond the ears of the absorbent article. As can be seen in Figure 12B, favorably the distal end 14 of each fastening tab 10 is folded onto its proximal end (no longer visible, 12) so that the male fastening elements (no longer visible, 22) are releasably engaged with the engaging-structure (no longer visible, 42). It will be appreciated from Figure 12B that upon the folding of the tabs in this exemplary embodiment, the ears 140 of the absorbent article 100 remain extended. Favorably for storage and/or packaging purposes, the ears of the article are also folded over towards the interior as shown in Figure 12C.

Absorbent articles including at least one fastening tab as described herein wherein the distal end of the fastening tab is folded onto the proximal end of the fastening tab so that the male fastening elements are releasably engaged with the engaging-structure are preferred for storage and/or product packaging purposes. Accordingly an advantageous method of manufacturing such an absorbent article, in particular a diaper or incontinence brief, comprises the steps of: a) providing an absorbent article comprising a back sheet, a top sheet, and an absorbent core therebetween; b) providing at least one fastening tab as described herein; c) affixing the at least one fastening tab with at least its proximal end to the absorbent article; and d) folding the distal end of the at least one fastening tab onto the proximal end of the at least one fastening tab so that the male fastening elements are releasably engaged with the engaging-structure.

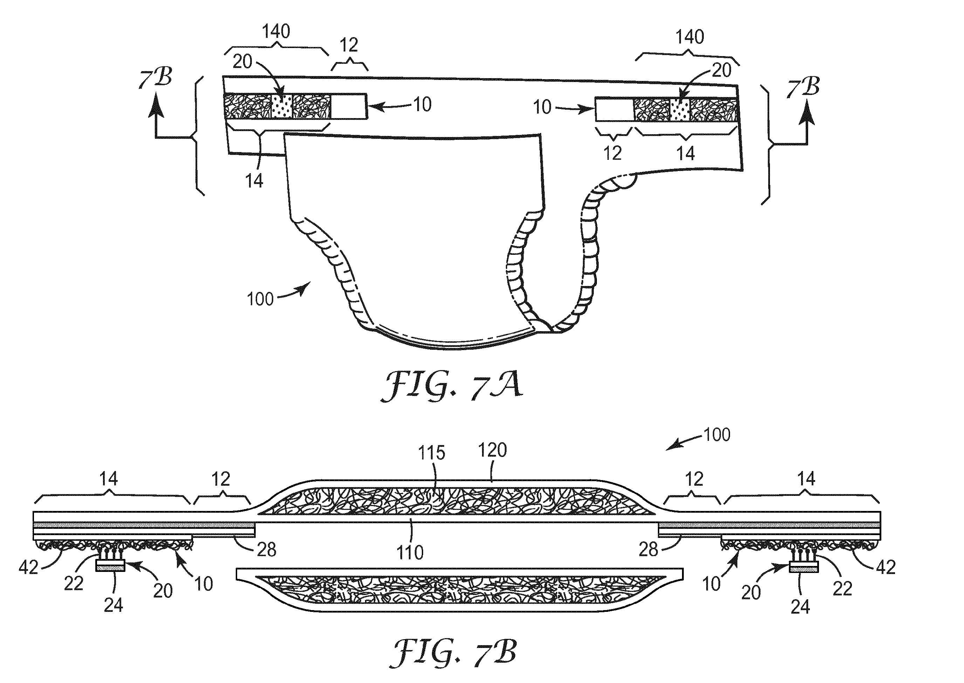

Methods of using such an absorbent article advantageously comprise the steps of: a) unfolding the distal end of the fastening tab so that the adhesive of the patch detaches from the release surface (e.g. low adhesion backside) and the male fastening elements of the patch remain engage with the engaging-structure, and b) closing the absorbent article by pressing the distal end of the fastening tab onto a respective portion of the absorbent article, thereby affixing the adhesive of patch (i.e. the first adhesive) to said respective portion of the absorbent article. This is best understood by making reference to Figures 7 through 9 which provide various views of the exemplary absorbent article shown in Figures 5 and 6 during various stages of use.

In particular Figures 7A and B represent isometric and cross sectional views, respectively, of the absorbent article in a configuration upon unfolding. Moreover, it can be seen in comparison to Figures 6A and B that the fastening tabs 10 as well as the ears 140 of the diaper or incontinence brief 100 are unfolded. Furthermore, it can be seen in comparison to Figures 5A and B that the male fastening element comprising patches 20 are now located in the distal ends 14 of the tabs 10. Referring to Figure 7B, it will be appreciated that upon unfolding, the adhesive 24 of the male fastening element comprising patch 20 detaches from the release surface 28, while the male fastening elements 22 of the patch remain engage with the engaging-structure 42, thus causing a locomotion of the patch from the proximal end 12 to the distal end 14 of the tab 10. It will also be recognized that the adhesive 24 of the male fastening element comprising patch is now exposed.

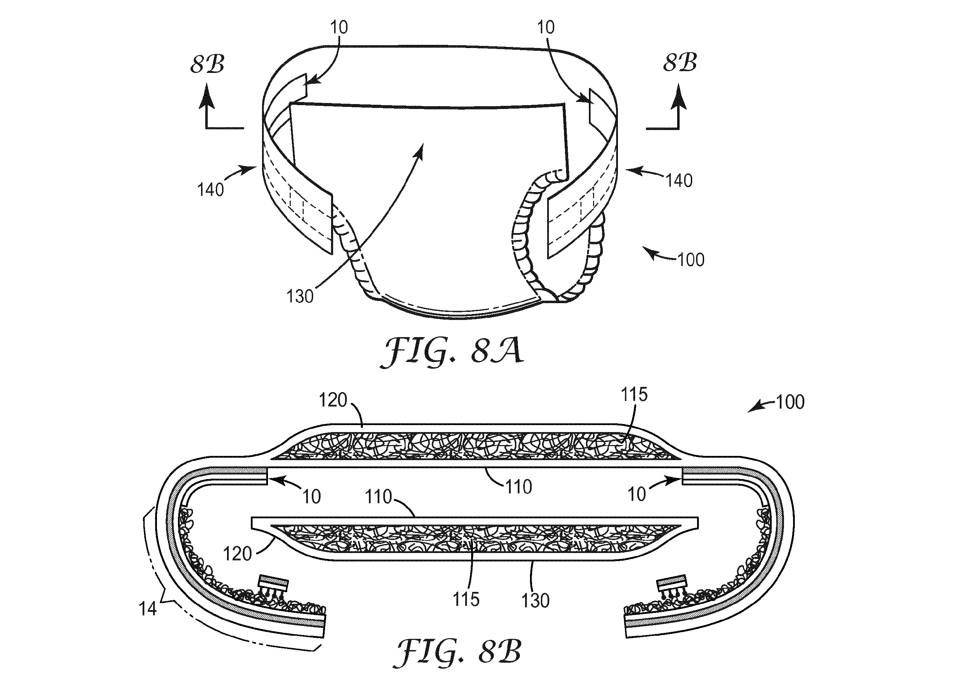

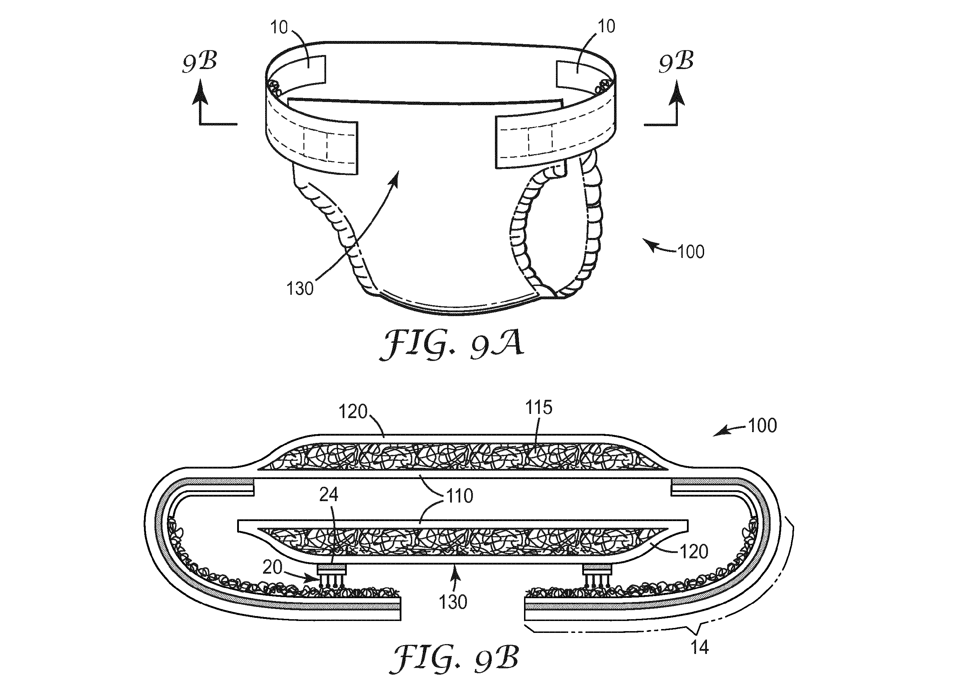

Figures 8A and B represent isometric and cross sectional views, respectively, of the absorbent article in a configuration between unfolding and the first closing of the absorbent article. In particular, it can be seen that the ears 140 of the diaper or incontinence brief 100 are shifted towards the front waist portion 130 in order to close the article and fasten the tabs 10 onto the back sheet 120 at that portion. Figure 9A and B represent isometric and cross sectional views, respectively, of the absorbent article in a closed or fastened configuration. In particular, the diaper or incontinence brief 100 is closed or fastened by pressing the distal end 14 of each fastening tab 10 onto the front waist portion 130 of the diaper or incontinence brief, thereby affixing the adhesive 24 of fastening element comprising patch 20 to said respective portion (in particular to the back sheet 120 found at that portion) of the diaper or incontinence brief. It will be appreciated that this closing/fastening can be advantageously achieved without having to provide either a landing zone or a back sheet being made of material suitable to engage the fastening elements of a mechanical fastening tab.

It will be further appreciated that the re-opening and re-closing of the absorbent article is advantageously possible. Favorable methods of use comprise the further step of: opening the absorbent article by pulling the distal end of the fastening tab so that the engaging-structure of the fastening tab disengages from the male fastening elements of the patch while the patch remains affixed to respective portion of absorbent article. Such favorable methods of use comprise more favorably the additional step of: re-closing the absorbent article by pressing the distal end of the fastening tab onto the patch so that the engaging-structure of fastening tab releasably engages the fastening elements of the patch.

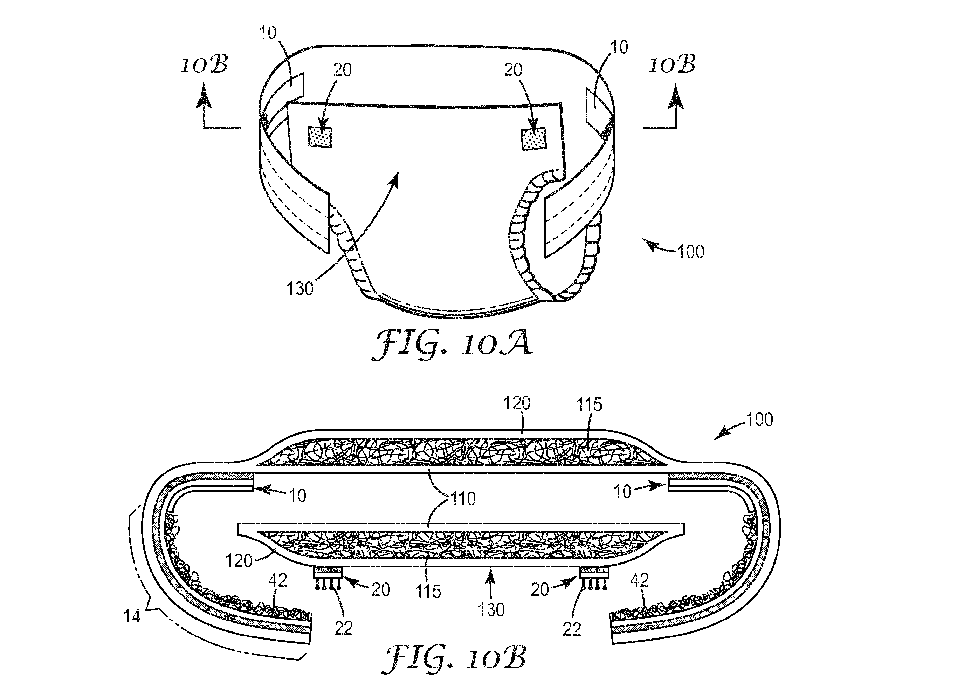

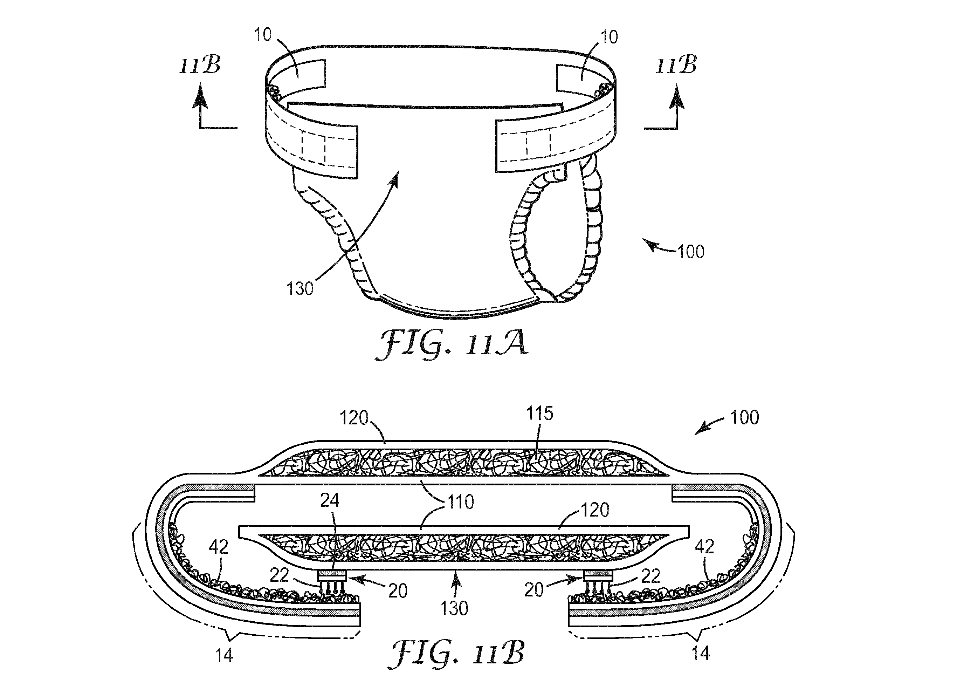

This is best understood by making reference to Figures 10 and 11. In particular Figures 10 and 11 represent isometric views (A) and cross-sectional views (B), respectively, of the exemplary absorbent article shown in Figures 5 to 9 where the absorbent article is shown in a configuration after opening and after re-closing of the absorbent article, respectively. It can be recognized from Figures 10A and B that in order to open the diaper or incontinence brief 100, the distal ends 14 of the tabs 10 are pulled away from the front waist portion 130 so that the engaging-structure 42 of fastening tab disengages from the male fastening elements 22 of the patch 20 while the patch remains affixed to respective portion of diaper on incontinence brief. It can be recognized from Figures 11A and B that in order to re-close the diaper or incontinence brief 100, the distal ends 14 of the tabs 10 are pressed onto fastening element comprising patch 20 at the front waist portion 130 so that the engaging-structure 42 of fastening tab releasably re-engages the male fastening elements 22 of the patch 20.

## Claims

1. A fastening tab for an absorbent article, the fastening tab comprising a proximal end and a distal end,
said proximal end comprising a backing including a first major surface and a second major surface, wherein said first major surface is provided with a release surface, wherein the fastening tab further comprises a mechanical fastening patch, said patch comprising on one side male fastening elements and on the opposite side an adhesive, wherein the patch is releasably attached to the release surface via the adhesive,
said distal end comprising a backing including a first major surface and a second major surface, wherein said first major surface has a structure or is provided with a structure that is adapted to be releasably engaged by said male fastening elements of the patch.

2. A fastening tab according to claim 1, wherein the proximal and distal ends of the tab are configured and arranged such that at least a portion of one end (in particular the distal end) can be folded onto the other end (in particular the proximal end) so that said male fastening elements can releasably engage said engaging-structure.

3. A fastening tab according to claim 1 or 2, wherein said fastening tab is configured and arranged, such that the force to disengage the male fastening elements from the engaging-structure is higher than the force to detach the adhesive of the fastening element patch from the release surface; and/or wherein the release surface is selected from the group consisting of a low surface energy film, a microstructured film, or a low adhesion backside coating.

4. A fastening tab according to any one of the preceding claims, wherein the adhesive of the patch is a first adhesive and the second major surface at the proximal end is provided at least in part with a second adhesive.

5. A fastening tab according to any one of the preceding claims, wherein the second major surface at the distal end is provided at least in part with a third adhesive.

6. A fastening tab according to claim 5 as dependent on claim 4, wherein the second and third adhesives are identical or different.

7. The fastening tab according to any of the preceding claims, whereby the backing of the proximal end and the backing of the distal end are either integral or two separate elements, said two separate elements being connected to another via a connection member.

8. A fastening tab according to any of the preceding claims, wherein said male fastening elements are selected from the group consisting of hook fasteners, mushroom-shaped fasteners, stem-shaped fasteners, cup-shaped fasteners, T-shaped fasteners and mixtures thereof.

9. A fastening tab according to any of the preceding claims, wherein said engaging-structure comprises a structure selected from the group consisting of nonwoven structures, loops, mushroom-shaped fasteners, cup-shaped fasteners, T-shaped fasteners and mixtures thereof.

10. A fastening tab according to any of the preceding claims, wherein the first adhesive is a pressure-sensitive adhesive.

11. A fastening tab according to any of the preceding claims, wherein the first adhesive has a 90° peel adhesion to a polyethylene test surface ha ving average surface roughness value Rₐ of about 1.4 µm and an average peak to valley height value R_{z} of about 12.5µm greater than 5 N/25 mm, in particular equal to or greater than 6 N/25 mm, more particularly equal to or greater than 7 N/25mm.

12. A fastening tab according to any of the preceding claims, wherein the first adhesive has a tack value to a polyethylene test surface has an average surface roughness value Rₐ of about 1.4 µm and an average peak to valley height value R_{z} of about 12.5µm equal to or greater than 5 N/25 mm, in particular equal to or greater than 6 N/25 mm, more particularly equal to or greater than 7 N/25mm.

13. A fastening tab according to any of the preceding claims, wherein relative to the transverse direction of the tab from the proximal end to the distal end, the width of the engaging-structure is at least 2 times greater than the width of the male fastening element comprising patch, in particular the width of the engaging-structure is at least 2.5 times greater than the width of the of male fastening element comprising patch, more particularly the width of the engaging-structure is at least 3 times greater than the width of the of male fastening element comprising patch.

14. A fastening tape comprising a plurality of fastening tabs according to any one of preceding claims, wherein the fastening tabs are arranged in an endless form on the tape so that individual fastening tabs can be provided from the fastening tape by cutting the tape across its width.

15. A roll of fastening tape comprising a plurality of fastening tabs according to claim 14.

16. A roll according to claim 15, wherein the proximal and distal ends of the fastening tabs are configured and arranged in the roll such that said male fastening elements are not engaging said engaging-structure; and/or wherein relative to the width of the tape as provided in the roll, the proximal and distal ends of the fastening tabs are positioned in two outer lateral regions of the rolled fastening tape.

17. A roll according to claim 15 or 16, wherein the male fastening elements include ends distal to the first adhesive, wherein in the roll, said ends are positioned towards the second major surface at the proximal end.

18. A roll according to claim 17, wherein the second major surface at the proximal end is provided at least in part with a second adhesive and said ends of the male fastening elements are in contact with said second adhesive.

19. A roll according to any one of claims 15 to 18, wherein the roll is free of a release liner between the male fastening elements and the second major surface at the proximal end; and/or wherein the roll is free of a release liner between the engaging-structure and the second major surface at the distal end.

20. An absorbent article comprising a back sheet, a top sheet and an absorbent core there between and at least one fastening tab according to any one of claims 1 to 13, wherein at least the proximal end of the at least one fastening tab is affixed to the absorbent article.

21. An absorbent article according to claim 20, wherein the absorbent article is a diaper or incontinence brief.

22. An absorbent article according to claim 21, wherein the at least one fastening tab is affixed to the rear waist portion of the diaper or incontinence brief, in particular to the inside of the rear waist portion.

23. An absorbent article according to any one of claims 20 to 22, wherein the absorbent article further comprises an ear, and wherein the at least one fastening tab is attached to the ear at least at a distal end portion of the ear, in particular to the inside of the ear at a distal end portion thereof.

24. An absorbent article according to any one of claims 20 to 23, wherein the distal end of the at least one fastening tab is folded onto the proximal end of the fastening tab so that the male fastening elements are releasably engaged with the engaging-structure.

25. A method of manufacturing an absorbent article, in particular a diaper or incontinence brief, comprising the steps of:
a) providing an absorbent article comprising a back sheet, a top sheet, and an absorbent core therebetween;
b) providing at least one fastening tab according to any one of claims 1 to 13;
c) attaching the at least one fastening tab with at least its proximal end to the absorbent article; and
d) folding the distal end of the at least one fastening tab onto the proximal end of the fastening tab so that the male fastening elements are releasably engaged with the engaging-structure.

26. A method of manufacturing according to claim 25, wherein the step b) includes the sub-steps of:
i) providing a roll of fastening tape according to any one of claims 15 to 19;
ii) unwinding tape from the roll; and
iii) cutting unwound tape across its width in order to provide at least one fastening tab.

27. A method of using an absorbent article according to claim 24, comprising the steps of:
a) unfolding the distal end of the at least one fastening tab so that the adhesive of the patch detaches from the release surface and the male fastening elements of the patch remain engage with the engaging-structure, and
b) closing the absorbent article by pressing the distal end of the at least one fastening tab onto a respective portion of the absorbent article, thereby adhering the adhesive of patch to said respective portion of the absorbent article.

28. A method according to claim 27, the method further comprising the step of:
c) opening the absorbent article by pulling the distal end of the at least one fastening tab so that the engaging-structure of the fastening tab disengages from the male fastening elements of the patch while the patch remains affixed to respective portion of absorbent article.

29. A method according to claim 28, the method further comprising the step of:
d) re-closing the absorbent article by pressing the distal end of the at least one fastening tab onto the patch so that the engaging-structure of fastening tab releasably engages the male fastening elements of the patch.

30. A method according to any one of claims 27 to 29, wherein the absorbent article is a diaper or incontinence brief and said respective portion of the absorbent article is a front waist portion of the diaper or incontinence brief, in particular the outside of the front waist portion of the diaper or incontinence brief.
